# EUROPEAN PATENT SPECIFICATION

(11) **EP 0 637 449 B1**
(45) Date of publication and mention of the grant of the patent: **07.01.1998**
(21) Application number: 94830306.0
(22) Date of filing: 22.06.1994
(51) Int. Cl.: A61K 31/205

(54) **Use of L-carnitine or acyl L-carnitines and valproate for treating seizure disorders**
Verwendung von L-Carnitine oder Acyl-L-Carnitinen und Valproat zur Behandlung von Anfallserkrankungen
Utilisation de L-carnitine ou acyl-L-carnitines et valproate pour le traitement des attaques brusques

(30) Priority: 06.07.1993 IT RM930441
(43) Date of publication of application: 08.02.1995
(73) Proprietor: Sigma-Tau Industrie Farmaceutiche Riunite S.p.A., 00144 Roma (IT)
(72) Inventor: Cavazza, Claudio, I-00186 Rome (IT)
(74) Representative: Fassi, Aldo, Dr.

(56) References cited:
- DE-A- 3 726 945
- EPILEPSIA vol. 28 , 1987 pages 373 - 377 T. SUGIMOTO ET AL. 'Hepatoxicity in rat following administration of valproic acid: Effect of L-carnitine supplementation'
- ANN. NEUROL vol. 24 , 1988 page 301 D. COULTER 'Prevention of hepatoxicity recurrence with valproate monotherapy and carnitine'
- INT. PEDIATR. vol. 5, no. 1 , 1990 pages 54 - 57 M. CASTRO-GAGO ET AL. 'Effects of valproic acid on the urea cycle and carnitine metabolism'
- PEDIATR. RES. vol. 31, no. 4PT1 , April 1992 page 419 J. HOLOWACH ET AL. 'Amelioration of adverse effects of valproic acid on ketogenesis and liver coenzyme A metabolism by cotreatment with pantothenate and carnitine in developing mice: Possible clinical significance'
- PHARMACOL. TOXICOL. vol. 72, no. 3 , March 1993 pages 145 - 147 M. MATSUOKA ET AL. 'Effects of L and D-carnitine on brain energy metabolites in mice given sublethal doses of ammonium acetate'
- BRAIN RES. vol. 567, no. 2 , 1991 pages 328 - 331 M. MATSUOKA ET AL. 'Suppression of neurotoxicity of ammonia by L-carnitine'

## Description

The present invention relates to a novel therapeutical use of L-carnitine, some acyl L-carnitines and their pharmacologically acceptable salts for treating seizure disorders (epilepsy).

More specifically, the present invention relates to the coordinated use of L-carnitine, acyl L-carnitines or their pharmacologically acceptable salts and valproate (i.e. a pharmacologically acceptable salt of valproic acid, such as sodium or magnesium valproate) for decreasing the seizure frequency in epileptic patients.

Within the scope of the present invention, by "co-ordinated use" of the aforesaid compounds it is meant indifferently both the co-administration, i.e. the substantially concomitant supplementation of L-carnitine or acyl L-carnitine or a pharmacologically acceptable salt thereof and valproate, as active ingredients, and the administration of a combination preparation containing a mixture of the aforesaid active ingredients, in addition to suitable excipients, if any.

L-carnitine supplementation concomitantly administered with valproate therapy in epileptic patients, particularly epileptic young children, has been already disclosed although the therapeutical effect aimed at does not allow the novel use, object of the present invention, to be in any way foreseen.

Valproate is a remarkably efficacious anticonvulsant; however, in some cases it can cause a severe or fatal hepatotoxicity. Valproate interferes with carnitine metabolism and this is the cause of valproate toxicity.

As known, carnitine performs two basic physiologic functions. First, it carries long-chain fatty acids across the inner mitochondrial membrane into the mitochondrion matrix where the beta-oxidation takes place. Secondly, carnitine helps to regulate the intramitochondrial ratio of acyl CoA to free CoA. Acyl carnitine transferase located on the inner wall of inner mitochondrial membrane favours the binding of carnitine to acylCoA forming acylcarnitine esters for transport out of the mitochondrion.

The same reaction releases and makes available the CoA within the mitochondrion. This carnitine function is essential since it removes excessive (and potentially toxic) short and medium chain fatty acids from the mitochondrion, maintaining sufficient free CoA within the mitochondrion to provide adequate cell energy metabolism.

Tissue (mitochondrial) carnitine deficiency affects the energy metabolism by hindering the beta-oxidation of long-chain fatty acids, allowing the accumulation of acyl CoA in mitochondria to occur, which results in lower amounts of free CoA to be available.

This endangers the activities of those tissues and organs, such as the muscular tissue and the brain, which are most heavily dependent on mitochondrial energy metabolism, and brings about the onset of the symptoms and signs of encephalopathy and myopathy.

Also liver dysfunctions can result from reduced mitochondrial metabolism in the liver and the release of hepatotoxic metabolites in the blood stream.

A number of causes may lead to even severe carnitine deficiencies in epileptic patients. The most frequent causes include inborn metabolic disorders, inappropriate dietary intake and medication with anticonvulsant drugs.

Among these drugs, valproate exerts a potent effect on carnitine-linked metabolism. Indeed, valproic acid (2-propylpentanoic acid), a short-chain fatty acid, combines with carnitine in mitochondria forming valproylcarnitine for transport out of mitochondria and excretion into urine.

In 1982, Othani et al (Othani et al., Carnitine deficiency and hyperammonemia associated with valproate therapy, J. Pediatr. 1982; 101: 782-785) were the first Authors who detected carnitine deficiency in valproate-treated patients.

Already in 1981, Haas et al (Haas R. et al., Inhibitory effects of sodium valproate on oxidative phosphorylation, Neurology 1981; 31: 1473-1476) had shown valproate toxicity towards mitochondria of rat liver in vitro, subsequently shown also in vivo (Sugimoto et al., Hepatotoxicity in rat following administration of valproic acid: effect of L-carnitine supplementation, Epilepsia 1987; 28: 373-377).

Furthermore, valproate can inhibit mitochondrial beta-oxidation of long-chain fatty acids via two mechanisms. The first is the formation of valproyl-CoA which sequesters free CoA thus decreasing its availability for fatty acid metabolism. The second, even more importantly, is the inhibition of the enzymes which play a role in beta-oxidation by some valproate metabolites, notably 4-en valproate.

It was furthermore found that the administration of anticonvulsants can cause severe to fatal hepatotoxicity, particularly in young children.

For this reason, some Authors (see e.g. David L. Coulter, Prevention of hepatotoxicity recurrence with valproate monotherapy and carnitine, [abstract] Ann. Neurol. 1988, 24: 301) have proposed a clinical protocol providing an antiepileptic valproate monotherapy with concomitant carnitine administration. This Author concludes: "These results suggest that children with previous hepatotoxic reactions to valproate may tolerate the drug without recurrence of hepatotoxicity when this protocol is used. Valproate monotherapy with carnitine supplementation could allow more children to benefit from this drug".

It has now been found that the co-ordinated use of L-carnitine or an acyl L-carnitine wherein the acyl group has 2-8, preferably 2-6, carbon atoms or their pharmacologically acceptable salts and a pharmacologically acceptable salt of valproic acid dramatically decreases the seizure frequency in epileptic patients.

This remarkable therapeutical result could not be anticipated on the ground of the aforesaid known efficacy of L-carnitine in decreasing valproate hepatotoxicity.

The present invention, therefore, relates to the use of L-carnitine or an acyl L-carnitine wherein the acyl group has 2-8, preferably 2-6, carbon atoms or their pharmacologically acceptable salts and a pharmacologically acceptable salt of valproic acid for producing a medicament for decreasing the seizure frequency in epileptic patients.

Preferably, the acyl L-carnitine is selected from acetyl, propionyl, butyryl, valeryl and isovaleryl L-carnitine.

The pharmacologically acceptable salt of L-carnitine and acyl L-carnitine is selected from chloride, bromide, orotate, acid aspartate, acid citrate, acid phosphate, fumarate and acid fumarate, lactate, maleate and acid maleate, acid oxalate, acid sulphate, glucosephosphate, tartrate and acid tartrate.

Among the pharmacologically acceptable salts, the respective inner salts are included.

Examples of pharmacologically acceptable salts of valproic acid are sodium valproate and magnesium valproate.

The present invention also provides a pharmaceutical composition (combination preparation) for decreasing the seizure frequency in epileptic patients which comprises an acyl-L-carnitine wherein the acyl group is a linear or branched acyl having 2-8, preferably 2-6, carbon atoms or a pharmacologically acceptable salt thereof and a pharmacologically acceptable salt of valproic acid as active ingredients.

Preferred examples of acyl L-carnitines, pharmacologically acceptable salts of L-carnitine, acyl L-carnitines and valproic acid salts are those previously indicated.

A suitable pharmaceutical composition in unit dosage form comprises an amount of acyl L-carnitine or a pharmaceutically acceptable salt thereof equivalent to 0.3 to about 0.5 g of L-carnitine and from 0.2 to 0.5 g of sodium valproate or magnesium valproate.

The efficacy of the co-ordinated use of L-carnitine (or acyl L-carnitines) and valproate for decreasing the seizure frequency in epileptic patients has been tested in several clinical trials. One of these trials is described in detail hereinbelow.

### CLINICAL TRIAL

Fortyfive patients, 20 females and 25 males, mean age 24.5±8.48 years, were included in the study.

The patients exhibited the following seizure types:
generalized tonic clonic
partial simple
partial complex
absence
atonic
tonic
Lennox Gastaux

Some patients had more than one seizure type.

All of the patients had been treated only with valproate for at least six months.

Subsequently, they were treated with both valproate and L-carnitine for at least one year.

At intervals L-carnitine activity was assessed on some parameters including seizure frequency, in comparison with the pre-treatment period (valproate only, no L-carnitine). The intervals selected for all the controls were: between the start to 6 months of L-carnitine therapy, 6 months of L-carnitine therapy, and L-carnitine therapy greater than 12 months.

Parameter assessment was not carried out at prefixed dates but within the 0-6, 6-12 and greater than 12 month periods in order to evaluate the seizure frequency over a sufficiently extended time period.

### SEIZURE FREQUENCY:

Following L-carnitine treatment, the seizure frequency decreased progressively and steadily with respect to the pre-treatment period.
- Pre-carnitine period vs greater than 12 months L-carnitine period, p<0.001;
- Start-6 months vs greater than 12 months L-carnitine period, p=0.0001;
- 6-12 months vs greater than 12 months L-carnitine period, p=0.0001.

The effect was most pronounced after the first 6 months of L-carnitine therapy and continued to enhance after 1 year of L-carnitine therapy. The effect appeared to be independent on valproate dosage and serum concentration.

## Claims

1. Use of L-carnitine or an acyl L-carnitine wherein the acyl group has 2-8, preferably 2-6, carbon atoms or their pharmacologically acceptable salts and a pharmacologically acceptable salt of valproic acid for producing a medicament for decreasing the seizure frequency in epileptic patients.

2. The use of claim 1 wherein the acyl L-carnitine is selected from acetyl, propionyl, butyryl, valeryl and isovaleryl L-carnitine.

3. The use of claims 1 or 2 wherein the pharmacologically acceptable salt of L-carnitine or acyl L-carnitine is selected from inner salt, chloride, bromide, orotate, acid aspartate, acid citrate, acid phosphate, fumarate and acid fumarate, lactate, maleate and acid maleate, acid oxalate, acid sulphate, glucose phosphate, tartrate and acid tartrate.

4. The use according to anyone of the preceding claims wherein the pharmacologically acceptable salt of valproic acid is selected from sodium valproate and magnesium valproate.

5. A pharmaceutical composition for decreasing the seizure frequency in epileptic patients which comprises an acyl L-carnitine wherein the acyl group is a linear or branched acyl having 2-8, preferably 2-6, carbon atoms or a pharmacologically acceptable salt thereof and a pharmacologically acceptable salt of valproic acid.

6. The pharmaceutical composition of claim 5 wherein the acyl L-carnitine is selected from acetyl, propionyl, butyryl, valeryl and isovaleryl L-carnitine and the pharmacologically acceptable salt of valproic acid is selected from sodium valproate and magnesium valproate.

7. The pharmaceutical composition of claims 5 or 6 in unit dosage form comprising an amount of acyl L-carnitine or a pharmacologically acceptable salt thereof equivalent to 0.3-0.5 g of L-carnitine and from 0.2 to 0.5 g of sodium valproate or magnesium valproate.

## Patentansprüche

1. Verwendung von L-Carnitin oder einem Acyl-L-carnitin, worin die Acylgruppe 2 bis 8, bevorzugt 2 bis 6 Kohlenstoffatome hat, oder der pharmakologisch akzeptablen Salze davon und eines pharmakologisch akzeptablen Salzes von Valproinsäure zur Herstellung eines Medikamentes zur Verminderung der Anfallshäufigkeit bei epileptischen Patienten.

2. Verwendung nach Anspruch 1, worin das Acyl-L-carnitin aus Acetyl-, Propionyl-, Butyryl-, Valeryl- und Isovaleryl-L-carnitin ausgewählt ist.

3. Verwendung nach Anspruch 1 oder 2, worin das pharmakologisch akzeptable Salz von L-Carnitin oder Acyl-L-carnitin aus dem inneren Salz, Chlorid, Bromid, Orotat, saurem Aspartat, saurem Citrat, saurem Phosphat, Fumarat und saurem Fumarat, Lactat, Maleat und saurem Maleat, saurem Oxalat, saurem Sulphat, Glucosephosphat, Tartrat und saurem Tartrat ausgewählt ist.

4. Verwendung nach einem der vorhergehenden Ansprüche, worin das pharmakologisch akzeptable Salz von Valproinsäure aus Natriumvalproat und Magnesiumvalproat ausgewählt ist.

5. Pharmazeutische Zusammensetzung zur Verminderung der Anfallshäufigkeit bei epileptischen Patienten, umfassend ein Acyl-L-carnitin, worin die Acylgruppe eine lineare oder verzweigte Acylgruppe mit 2 bis 8, bevorzugt 2 bis 6 Kohlenstoffatomen ist, oder ein pharmakologisch akzeptables Salz davon und ein pharmakologisch akzeptables Salz von Valproinsäure.

6. Pharmazeutische Zusammenseetzung nach Anspruch 5, worin das Acyl-L-carnitin aus Acetyl-, Propionyl-, Butyryl-, Valeryl- und Isovaleryl-L-carnitin und das pharmakologisch akzeptable Salz von Valproinsäure aus Natriumvalproat und Magnesiumvalproat ausgewählt ist.

7. Pharmazeutische Zusammensetzung nach Anspruch 5 oder 6 in Einheitsdosierungsform, umfassend eine Menge aus Acyl-L-carnitin oder einem pharmakologisch akzeptblen Salz davon, das 0,3 bis 0,5 g L-Carnitin äquivalent ist, und von 0,2 bis 0,5 g Natriumvalproat oder Magnesiumvalproat.

## Revendications

1. Utilisation de L-carnitine ou d'une acyl-L-carnitine dont le groupe acyle compte 2 à 8, de préférence 2 à 6, atomes de carbone, ou d'un de leurs sels pharmacologiquement acceptables, et d'un sel pharmacologiquement acceptable d'acide valproïque pour produire un médicament destiné à diminuer la fréquence des crises chez des sujets épileptiques.

2. Utilisation de la revendication 1, dans laquelle l'acyl-L-carnitine est choisie parmi l'acétyl-, la propionyl-, la butyryl-, la valéryl- et l'isovaléryl-L-carnitines.

3. Utilisation selon les revendications 1 ou 2, dans laquelle le sel pharmacologiquement acceptable de L-carnitine ou d'acyl-carnitine est choisi parmi le sel interne, le chlorure, le bromure, l'orotate, l'aspartate acide, le citrate acide, le phosphate acide, le fumarate et le fumarate acide, le lactate, le maléate et le maléate acide, l'oxalate acide, le sulfate acide, le glucose-phosphate, le tartrate et le tartrate acide.

4. Utilisation selon l'une quelconque des revendications précédentes, dans laquelle le sel pharmacologiquement acceptable d'acide valproïque est choisi parmi le valproate de sodium et le valproate de magnésium.

5. Composition pharmaceutique destinée à diminuer la fréquence des crises chez des sujets épileptiques, qui comprend une acyl-L-carnitine dont le groupe acyle est un groupe acyle linéaire ou ramifié ayant 2 à 8, de préférence 2 à 6, atomes de carbone ou un sel pharmacologiquement acceptable de celle-ci, et un sel pharmacologiquement acceptable d'acide valproïque.

6. Composition pharmaceutique selon la revendication 5, dans laquelle l'acyl-L-carnitine est choisie parmi l'acétyl-, la propionyl-, la butyryl-, la valéryl- et l'isovaléryl-L-carnitines et le sel pharmacologiquement acceptable d'acide valproïque est choisi parmi le valproate de sodium et le valproate de magnésium.

7. Composition pharmaceutique selon les revendications 5 ou 6, sous une forme posologique unitaire comprenant une quantité d'acyl-L-carnitine ou d'un sel pharmaceutiquement acceptable de celle-ci qui est équivalente à 0,3 à environ 0,5 g de L-carnitine et 0,2 à 0,5 g de valproate de sodium ou de valproate de magnésium.
